# EUROPÄISCHE PATENTSCHRIFT

(11) **EP 0 722 925 B1**
(45) Veröffentlichungstag und Bekanntmachung des Hinweises auf die Patenterteilung: **28.10.1998**
(21) Anmeldenummer: 96100052.8
(22) Anmeldetag: 04.01.1996
(51) Int. Cl.: C07C 57/04, C07C 51/44

(54) **Verfahren der rektifikativen Abtrennung von (Meth)acrylsäure aus einem (Meth)acrylsäure enthaltenden Gemisch**
Process for rectificative separation of (meth)acrylic acid from the (meth)acrylic acid containing mixture
Procédé de séparation rectificative d'acide (méth)acrylique à partir d'un mélange renferment d'acide (méth)acrylique

(30) Priorität: 18.01.1995 DE 19501325
(43) Veröffentlichungstag der Anmeldung: 24.07.1996
(73) Patentinhaber: BASF AKTIENGESELLSCHAFT, 67056 Ludwigshafen (DE)
(72) Erfinder: Herbst, Holger, Dr., D-67227 Frankenthal (DE); Nestler, Gerhard, Dr., D-67061 Ludwigshafen (DE); Hammon, Ulrich, Dr., D-68165 Mannheim (DE)

(56) Entgegenhaltungen:
- WO-A-86/00236
- DE-A- 4 101 879
- DE-A- 4 308 087
- US-A- 2 147 306

## Beschreibung

Die vorliegende Erfindung betrifft ein neues Verfahren der rektifikativen Abtrennung von (Meth)acrylsäure aus einem (Meth)acrylsäure enthaltenden Gemisch, bei dem die (Meth)acrylsäure oberhalb der Stelle, bei der das die (Meth)acrylsäure enthaltende Flüssigkeitsgemisch in die Rektifikationskolonne eintritt, aus der Rektifikationskolonne entnommen wird, und bei dem man wenigstens an einer Stelle der Rektifikationskolonne die in derselben absteigende Rücklaufflüssigkeit aus der Rektifikationskolonne herausführt.

(Meth)acrylsäure wird als verkürzte Schreibweise verwendet und steht für Acrylsäure oder Methacrylsäure.

(Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich.

Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 4 405 059, EP-A 253 409, EP-A 92 097, DE-A 44 31 957 und DE-A 44 31 949).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine (Meth)acrylsäure sondern ein Reaktionsgemisch erhalten, das im wesentlichen (Meth)acrylsäure, die inerten Verdünnungsgase und Nebenprodukte enthält, aus welchem die (Meth)acrylsäure abgetrennt werden muß.

Neben von (Meth)acrylsäure vergleichsweise einfach zu entfernenden und bei Folgeverwendungen der (Meth)acrylsäure weniger störenden Nebenprodukten wie z.B. Essigsäure, enthält das Reaktionsgasgemisch insbesondere auch mit (Meth)acrylsäure eng verwandte und daher von (Meth)acrylsäure schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzlich gegebenenfalls Maleinsäureanhydrid (bezogen auf die im Reaktionsgasgemisch enthaltene Menge an (Meth)acrylsäure beträgt die Gesamtmenge dieser, bei Folgeverwendungen häufig erheblich störenden, Nebenkomponenten in der Regel ≤ 2 Gew.-%).

Üblicherweise erfolgt die Abtrennung der (Meth)acrylsäure aus dem Reaktionsgasgemisch über extraktive und rektifikative Trennverfahren, wobei die (Meth)acrylsäure bei letzteren in der Regel oberhalb der Stelle, bei der das die (Meth)acrylsäure enthaltende Gemisch in die Rektifikationskolonne eintritt, aus der Rektifikationskolonne entnommen wird (z.B. über Kopfentnahme oder durch Seitenabzug).

Die DE-A 44 36 243 betrifft z.B. ein Verfahren zur Abtrennung von (Meth)acrylsäure aus dem Reaktionsgasgemisch der katalylischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt, dem in der Absorptionskolonne in natürlicher Weise erfolgenden Absorptionsprozeß einen Rektifikationsprozeß überlagert, indem man der Absorptionskolonne eine über ihre, aufgrund ihres Kontaktes mit der Umgebungstemperatur erfolgende, natürliche Energieabgabe hinausgehende Energiemenge entzieht, und aus dem (Meth)acrylsäure und das Absorptionsmittel (Absorbens) als Hauptbestandteile sowie niedere Aldehyde und gegebenenfalls Maleinsäureanhydrid als Nebenbestandteile enthaltenden Flüssigkeitsablauf der Absorptionskolonne (Absorbat) die (Meth)acrylsäure rektifikativ über Seitenabzug (oberhalb der Zufuhrstelle in die Rektifikationskolonne) abtrennt. Die dabei erhältliche (Meth)acrylsäure wird als Roh- (Meth)acrylsäure bezeichnet. Sie weist in der Regel eine Reinheit > 98 Gew.-% auf, wobei sich die Verunreinigungen insbesondere aus den benannten niederen Aldehyden und gegebenenfalls Maleinsäureanhydrid rekrutieren, wohingegen die Trennung der (Meth)acrylsäure von der hochsiedenden inerten organischen Absorptionsflüssigkeit im wesentlichen quantitativ erfolgt ist.

Als hochsiedende inerte hydrophobe organische Flüssigkeit (Absorbens) faßt die DE-A 44 36 243 dabei alle diejenigen Flüssigkeiten zusammen, deren Siedetemperatur bei Normaldruck (1 atm) oberhalb der Siedetemperatur der (Meth)acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden.

Aus der DE-PS 2 136 396 und der DE-A 43 08 087 ist ebenfalls bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen. Das Verfahren wird im wesentlichen so durchgeführt, daß man das Reaktionsgasgemisch in einer konventionellen Absorptionskolonne zu der absteigenden Absorptionsflüssigkeit im Gegenstrom führt, danach in einer Desorptionskolonne aus den im wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponenten weitgehend entfernt und anschließend den Acrylsäure und das Absorbens als Hauptbestandteile sowie niedere Aldehyde und gegebenenfalls Maleinsäureanhydrid als Nebenbestandteile enthaltenden Flüssigkeitsablauf der Desorptionskolonne zur Abtrennung von Roh-Acrylsäure über Kopf in einer Rektifikationskolonne rektifikativ behandelt.

Die EP-B 102 642, die GB-PS 1 346 737 und die DE-B 2 207 184 betreffen die rektifikative Abtrennung von Rein-(Meth)acrylsäure aus Roh-(Meth)acrylsäure, die dadurch begünstigt wird, daß man der Roh- (Meth)acrylsäure Verbindungen zusetzt, die eine -NH₂-Gruppe aufweisen. Die primären Amine binden die als Verunreinigungen enthaltenen Aldehyde in hohem Ausmaß unter Ausbildung hochsiedender Verbindungen, von denen Rein-(Meth)acrylsäure z.B. über Kopf in einer Rektifikationskolonne in hoher Reinheit abgetrennt werden kann.

Aus der DE-A-4 101 879 ist die rektifikative Abtrennung von Acrylsäure aus einem Flüssigkeitsgemisch bekannt, bei dem man zur Vermeidung der Bildung von Rückständen wenigstens an einer Stelle der Rektifikationskolonne die in derselben absteigende Rücklaufflüssigkeit aus der Rektifikationskolonne herausführt. Die US-A-2 147 306 betrifft die rektifikative Abtrennung von Fettsäuren unter Entnahme eines Seitenabzugs, der nach Reinigung wieder in die Rektifikationskolonne zurückgeführt wird.

Nachteilig an allen rektifikativen Trennverfahren, bei denen (Meth)acrylsäure oberhalb der Stelle, bei der das die (Meth)acrylsäure enthaltende Gemisch in die Rektifikationskolonne eintritt, aus der Rektifikationskolonne entnommen wird, ist, daß sich während der rektifikativen Abtrennung, auch bei Mitverwendung von Polymerisationsinhibitoren wie Luft, Hydrochinon, Hydrochinonmonomethylether, Paranitrosophenol, Paramethoxyphenol und/oder Phenothiazin, die Rektifikationsvorrichtungen (insbesondere die Verdampferoberfläche) relativ rasch mit Belag bedecken, weshalb die üblicherweise kontinuierlich durchgeführte Rektifikation von Zeit zu Zeit zum Zwecke der Entfernung der Belagsbildung unterbrochen werden muß. Dies ist auch dann der Fall, wenn das die (Meth)acrylsäure enthaltende Ausgangsgemisch keine niederen Aldehyde umfaßt. Ein Beisein letzterer verstärkt jedoch in der Regel die Belagsbildung.

Die Aufgabe der vorliegenden Erfindung bestand daher darin, ein neues Verfahren der rektifikativen Abtrennung von (Meth)acrylsäure aus einem (Meth)acrylsäure enthaltenden Gemisch, bei dem die (Meth)acrylsäure oberhalb der Stelle, bei der das die (Meth)acrylsäure enthaltende Flüssigkeitsgemisch in die Rektifikationskolonne eintritt, aus der Rektifikationskolonne entnommen wird, und bei dem man wenigstens an einer Stelle der Rektifikationskolonne die in derselben absteigende Rücklaufflüssigkeit aus der Rektifikationskolonne herausführt, zur Verfügung zu Stellen, bei dem die Belagsbildung auf den Rektifikationsvorrichtungen (insbesondere auf der Verdampferoberfläche) reduziert ist.

Demgemäß wurde ein Verfahren der rektifikativen Abtrennung von (Meth)acrylsäure aus einem (Meth)acrylsäure enthaltenden Gemisch, bei dem die (Meth)acrylsäure oberhalb der Stelle, bei der das die (Meth)acrylsäure enthaltende Flüssigkeitsgemisch in die Rektifikationskolonne eintritt, aus der Rektifikationskolonne entnommen wird, und bei dem man wenigstens an einer Stelle der Rektifikationskolonne die in derselben absteigende Rücklaufflüssigkeit aus der Rektifikationskolonne herausführt, gefunden, dadurch gekennzeichnet, daß man die in der aus der Rektifikationskolonne herausgeführten absteigenden Rücklaufflüssigkeit enthaltene oligo- und/oder polymerisierte (Meth)acrylsäure abtrennt und anschließend die Rücklaufflüssigkeit wieder in die Rektifikationskolonne zurückführt.

Vorstehende Verfahrensweise ist das Ergebnis ausgedehnter Forschungsarbeit, im Rahmen derer nachfolgende Zusammenhänge gefunden wurden.

Zunächst wurde festgestellt, daß trotz des Beiseins von Polymerisationsinhibitoren längs der Rektifikationskolonne in einem gewissen Ausmaß radikalische Oligo- und/oder Polymerisation der (Meth)acrylsäure erfolgt. Die dabei entstehenden (Meth)acrylsäureoligo- und/oder -polymerisate weisen einen im Vergleich zur (Meth)acrylsäure erhöhten Siedepunkt auf, weshalb sie sich bei Rektifikationen, bei denen die (Meth)acrylsäure oberhalb der Stelle, bei der das die (Meth)acrylsäure enthaltende Flüssigkeitsgemisch in die Rektifikationskolonne eintritt, aus der Rektifikationskolonne entnommen wird, zum Verdampfer hin anreichern. Des weiteren wurde gefunden, daß Oligo- und/oder Polymerisate der (Meth)acrylsäure in (Meth)acrylsäure eine erhöhte molekulare und/ oder kolloidale Löslichkeit aufweisen. Da die in der Rektifikationskolonne absteigende Rücklaufflüssigkeit von der Entnahmestelle der (Meth)acrylsäure zum Verdampfer hin zunehmend an (Meth)acrylsäure verarmt, sinkt somit normalerweise die molekulare und/oder kolloidale Löslichkeit der (Meth)acrylsäureoligo- und/oder -polymerisate in der Rücklaufflüssigkeit zum Verdampfer hin. Gleichzeitig reichern sich die (Meth)acrylsäureoligo- und/oder -polymerisate, wie vorstehend beschrieben, in dieser Richtung an und erhöhen ihren Polymerisationsgrad bis ihre Löslichkeit in der Rücklaufflüssigkeit überschritten wird und sie sich als Belag auf den Rektifikationsvorrichtungen niederschlagen und adsorptiv auf selbigen festhaften. Letzteres gilt insbesondere für die Verdampferoberfläche, auf welcher lokal infolge des Verdampfens der (Meth)acrylsäure eine besonders ausgeprägte Entreicherung an (Meth)acrylsäure einhergeht.

Enthält das der Rektifikationskolonne zugeführte Einsatzgemisch Aldehyde, überlagert sich insbesondere im unteren Teil der Rektifikationskolonne aufgrund der dort vorherrschenden erhöhten Temperaturen auf die Aldehyde zurückzuführende Harzbildung, die ebenfalls zu Belagsbildung führt.

Gemäß vorstehenden Ausführungen ist leicht verständlich, daß ein Herausführen der in der Rektifikationskolonne absteigenden Rücklaufflüssigkeit an wenigstens einer Stelle (der erfindungsgemäße Verfahrensschritt kann längs der Rektifikationskolonne selbstredend mehrfach angewendet werden) der Rektifikationskolonne sowie ein sich daran anschließendes Abtrennen von in der herausgeführten Rücklaufflüssigkeit enthaltener oligo- und/oder polymerisierter (Meth)acrylsäure und ein darauffolgendes Zurückführen der gereinigten Rücklaufflüssigkeit in die Rektifikationskolonne in selbiger die Belagsbildung signifikant mindert und damit verlängerte Betriebsdauern der Rektifikationskolonne bedingt.

Zweckmäßigerweise wird man das erfindungsgemäße Verfahren in einer aus Abtriebsteil und Verstärkerteil bestehenden Rektifikationskolonne durchführen. D.h. die Einspeisung des rektifikativ aufzutrennenden Einsatzgemisches erfolgt vorzugsweise nicht im Sumpf, sondern an einer längs der Kolonne gelegenen Eintrittstelle. Der unterhalb dieser Eintrittsstelle gelegene Teil der Rektifikationskolonne wird als Abtriebsteil und der oberhalb dieser Eintrittstelle gelegene Teil der Rektifikationskolonne wird als Verstärkerteil bezeichnet. Mit Vorzug wird man in diesem Fall die in der Rektifikationskolonne absteigende Rücklaufflüssigkeit unmittelbar oberhalb der Eintrittstelle des Einsatzgemisches in die Rektifikationskolonne (d.h. am unteren Ende des Verstärkungsteils der Rektifikationskolonne) aus selbiger herausführen. Die Rückführung erfolgt dann zweckmäßigerweise unmittelbar unterhalb der vorgenannten Eintrittstelle (d.h. am oberen Ende des Abtriebteils der Rektifikationskolonne).

Als mögliche Verfahren zum Abtrennen der oligo- und/oder polymerisierten (Meth)acrylsäure von der aus der Rektifikationskolonne herausgeführten Rücklaufflüssigkeit kommen insbesondere die verfahren der Ultrafiltration (Nanofiltration) und/oder des Ultrazentrifugierens in Betracht. Beide genannten Verfahren sind auch im Rahmen einer rektifikativen Abtrennung von Rein-(Meth)acrylsäure aus Roh-(Meth)acrylsäure anwendbar, da sowohl die räumliche Ausdehnung als auch die Massendichte von oligo- und/oder polymerisierter (Meth)acrylsäure von der entsprechenden Eigenschaft der monomeren (Meth)acrylsäure ausreichend verschieden ist. Der Unterschied in der Massendichte fußt insbesondere auf dem erhöhten Raumbedarf der Elektronenwolke einer ungesättigte Doppelbindung (beispielsweise beträgt die Massendichte von polymerisierter Acrylsäure bei 25°C und 1 atm 1,54 g/cm³, wohingegen unter denselben Bedingungen die Massendichte der monomeren Acrylsäure 1,05 g/cm³ beträgt; im Fall der (Meth)acrylsäure beträgt der Unterschied der Massendichten unter den genannten Bedingungen 1,45 g/cm³ zu 1,01 g/cm³; vgl. z.B. Ullmann's Encyclopedia of Industrial Chemistry, fifth edition, 1992, VCH Verlagsgesellschaft, Weinheim, S. 169 sowie D'ANS-LAX, Taschenbuch für Chemiker und Physiker, Dritte Auflage, Springer Verlag, 1964, Band II, Organische Verbindungen). Infolge der erhöhten Massendichte reichert sich die kolloidal gelöste oligo- und/oder polymerisierte (Meth)acrylsäure am Mantel der Ultrazentrifuge an und kann von selbigem kontinuierlich abgeschält werden. Selbstverständlich können zur Abtrennung auch chromatographische Verfahren angewendet werden. Eine andere allgemein anwendbare Trennweise besteht darin, die flüchtigen Bestandteile der entnommenen Rücklaufflüssigkeit unter reduziertem Druck zu verdampfen und die dabei gebildete Dampfphase als solche oder nach Kondensation in die Rektifikationskolonne zurückzuführen.

In besonders einfacher und effizienter Weise läßt sich die erfindungsgemäße Abtrennung der oligo- und/oder polymerisierten (Meth)acrylsäure aus der aus der Rektifikationskolonne herausgeführten Rücklaufflüssigkeit dann verwirklichen, wenn das rektifikative Verfahren die Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch betrifft, wie es z.B. im Rahmen der Abtrennung von (Meth)acrylsäure aus dem Reaktionsgasgemisch der katalytischen Gasphasenoxidation gemäß den in der DE-A 44 36 243, der DE-PS 2 136 396 und der DE-A 4 308 087 beschriebenen Verfahrensweisen auftritt. D.h. wenn das (Meth)acrylsäure und eine höher als (Meth)acrylsäure siedende inerte hydrophobe organische Flüssigkeit als Hauptbestandteile sowie niedere Aldehyde als Nebenbestandteile enthaltende Einsatzgemisch für das erfindungsgemäße Verfahren z.B. aus den Reaktionsgasgemischen der katalytischen Gasphasenoxidation als Flüssigkeitsablauf einer Gegenstromabsorption mit anschließender Desorption durch Abstreifen gemäß der DE-PS 2 136 396 oder der DE-A 4 308 087 oder als Flüssigkeitsablauf einer Gegenstromabsorption mit überlagerter Rektifikation gemäß der DE-A 4 436 243 erhalten wurde. Unter hochsiedender inerter hydrophober organischer Flüssigkeit sind hier solche Flüssigkeiten zu verstehen, deren Siedepunkt bei Normaldruck (1 atm) oberhalb der Siedetemperatur der (Meth)acrylsäure liegt und in denen die Löslichkeit (Gew.-%, bezogen auf das Gewicht der Lösung) von (Meth)acrylsäureoligo- und/oder -polymerisaten bei 25°C und 1 atm geringer als in reiner (Meth)acrylsäure ist.

Insbesondere sind es solche hochsiedenden organischen Flüssigkeiten, die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Im engeren Sinn umfaßt der Begriff hier die hochsiedenden organischen Absorptionsflüssigkeiten, die in der DE-PS 2 136 396, der DE-A 4 308 087 sowie der DE-A 4 436 243 empfohlen werden. Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl, oder Mischungen der vorgenannten Flüssigkeiten wie z.B. ein Gemisch aus 70 bis 75 Gew.-% Diphenylether und 25 bis 30 Gew.-% Diphenyl. Eine günstige hochsiedende hydrophobe organische Absorptionsflüssigkeit ist auch ein Gemisch, bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat.

Im Fall der Methacrylsäure kann dabei die gasphasenkatalytisch oxidative Herstellung z.B. ausgehend von Methacrolein erfolgt sein, das seinerseits durch gasphasenkatalytische Oxidation von tert.-Butanol, iso-Butan oder iso-Buten oder durch Umsetzung von Formaldehyd mit Propionaldehyd gemäß der EP-B 92 097 oder der EP-B 58 927 erhalten worden sein kann. Häufig erfolgt die gasphasenkatalytische Oxidation des tert.-Butanols, iso-Butans oder iso-Butens unter Verwendung einer katalytisch aktiven Masse der allgemeinen Formel I

Mo₁₂BiₐFe_{b}X_{c}¹X_{d}²Xₑ³X_{f}⁴Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 30,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente in I bestimmt wird,

bei Temperaturen von 300 bis 400°C und, vom speziellen Temperaturverlauf abgesehen, ansonsten gemäß den Bedingungen der DE-A 4 023 239. Das dabei anfallende Methacrolein wird in der Regel ohne Zwischenreinigung zur Weiteroxidation verwendet. Dabei kann die gasphasenkatalytische Oxidation des Methacroleins, vom speziellen Temperaturverlauf abgesehen, gemäß den Ausführungen der DE-A 4 132 263 bei Temperaturen von 200 bis 350°C bzw. gemäß der DE-A 4 132 684 bei Temperaturen von 250 bis 400°C erfolgen. Insbesondere können dabei die in der DE-A 4 022 212 aufgeführten Multimetalloxidkatalysatoren angewendet werden.

Im Fall von Acrylsäure kann die gasphasenkatalytisch oxidative Herstellung z.B. einstufig ausgehend von Acrolein oder zweistufig ausgehend von Propylen über Acrolein erfolgt sein. Als Multimetalloxidkatalysatoren kommen dabei für die katalytische Gasphasenoxidation des Propylens insbesondere solche der allgemeinen Formel II

Mo₁₂BiₐFe_{b}X_{c}¹X_{d}²Xₑ³X_{f}⁴Oₙ (II),

in der die Variablen nachfolgende Bedeutung aufweisen:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente bestimmt wird,
und für die katalytische Gasphasenoxidation des Acroleins insbesondere solche der allgemeinen Formel III

Mo₁₂VₐW_{b}Cu_{c}Ni_{d}Xₑ¹X_{f}²X_{g}³Xₕ⁴Xᵢ⁵Oₙ (III)

in der die Variablen nachfolgende Bedeutung aufweisen:
- X¹: ein oder mehrere Alkalimetalle,
- X²: ein oder mehrere Erdalkalimetalle,
- X³: Chrom, Mangan, Cer und/oder Niob,
- X⁴: Antimon und/oder Wismut,
- X⁵: Silicium, Aluminium, Titan und/oder Zirkonium,
- a: 1 bis 6,
- b: 0,2 bis 4,
- c: 0,5 bis 6,
- d: 0,2 bis 6,
- e: 0 bis 2,
- f: 0 bis 3,
- g: 0 bis 5,
- h: 0 bis 40,
- i: 0 bis 40 und
- n: eine ganze Zahl, die durch die Wertigkeit und Häufigkeit der von Sauerstoff verschiedenen Elemente bestimmt wird,
in Betracht. Die Reaktionsgase der ersten Oxidationsstufe werden üblicherweise ohne Zwischenreinigung der zweiten Oxidationsstufe zugeführt. Die üblicherweise angewendeten Reaktionsbedingungen können z.B. der DE-A 431 957 sowie der DE-A 4 431 949 entnommen werden.

In der Regel enthält ein solches, vorstehend beschriebenes, im wesentlichen aus (Meth)acrylsäure und einer höher als (Meth)acrylsäure siedenden inerten hydrophoben organischen Flüssigkeit als Hauptbestandteilen sowie niederen Aldehyden als Nebenbestandteilen bestehendes Gemisch, 5 bis 25, meist 5 bis 15 Gew.-% (Meth)acrylsäure.

Da die rektifikative Abtrennung von (Meth)acrylsäure über Kopf- oder Seitenabzug der Rektifikationskolonne vorzugsweise bei reduziertem Druck erfolgt (zweckmäßigerweise bei einem Kopfdruck ≤ 500 mbar, üblicherweise 10 bis 500 mbar, häufig 10 bis 200 mbar und vorzugsweise 10 bis 100 mbar; in entsprechender Weise betragen die Sumpftemperaturen in der Regel 100 bis 230°C) und (Meth)acrylsäureoligo- und/oder -polymerisate in den zu verwendenden hochsiedenden inerten hydrophoben organischen Flüssigkeiten eine vergleichsweise reduzierte molekulare und/oder kolloidale Löslichkeit aufweisen, eröffnet sich im Fall der rektifikativen Abtrennung von (Meth)acrylsäure aus einem im wesentlichen aus (Meth)acrylsäure und einer höher als (Meth)acrylsäure siedenden inerten hydrophoben organischen Flüssigkeit als Hauptbestandteile sowie niederen Aldehyden als Nebenbestandteilen bestehenden Gemisch, nachfolgende einfache Möglichkeit der erfindungsgemäßen Abtrennung der oligo- und/oder polymerisierten (Meth)acrylsäure von der aus der Rektifikationskolonne herausgeführten Rücklaufflüssigkeit.

Das die rektifikativ abzutrennende (Meth)acrylsäure und die höher als (Meth)acrylsäure siedende inerte hydrophobe organische Flüssigkeit umfassende Gemisch wird dabei der aus Abtriebsteil und Verstärkerteil bestehenden Rektifikationskolonne nicht mehr unmittelbar zugeführt. Vielmehr wird das Einsatzgemisch zunächst in einen Verweilzeitbehälter geführt und erst nach einer gewissen Verweilzeit in selbigem (zweckmäßigerweise 0,1 bis 3 h) z.B. über einen unten angebrachten Ablauf der Rektifikationskolonne zugeführt (diese Zufuhrstelle definiert dann Verstärker- und Abtriebteil).

Der Verweilzeitbehälter wird darüber hinaus, z.B. durch Aufsetzen eines Brüdenrohres, mit dem Verstärkerteil der Rektifikationskolonne (normalerweise unmittelbar oberhalb der untersten theoretischen Trennstufe des Verstärkerteils) verbunden, was im zwischengeschalteten Verweilzeitbehälter einen entsprechenden Unterdruck erzeugt. Zum Beispiel durch Einbau eines Sammelbodens am unteren Ende des Verstärkerteils der Rektifikationskolonne wird der direkte Abstieg der Rücklaufflüssigkeit aus dem Verstärkerteil der Rektifikationskolonne in den Abtriebsteil der Rektifikationskolonne im wesentlichen unterbunden, die Rücklaufflüssigkeit aus der Rektifikationskolonne herausgeführt und, zweckmäßigerweise über eine getauchte Zuführung, dem Verweilzeitbehälter zugeführt. Ferner wird der Verweilzeitbehälter zweckmäßig so beheizt, daß das darin enthaltene Flüssigkeitsgemisch am Sieden gehalten wird.

D.h. der Verweilzeitbehälter hat die Funktion eines Verdampfers, wobei der (Meth)acrylsäure angereichert aufweisende Dampf dem Verstärkerteil der Rektifikationskolonne und die (Meth)acrylsäure entreichert aufweisende Sumpfflüssigkeit dem Abtriebsteil der Rektifikationskolonne zugeführt wird. Entsprechend kann der Verweilzeitbehälter beispielsweise als Verdampfer mit Selbstumlauf, als Verdampfer mit Zwangsumlauf, als Entspannungsverdampfer, als Dünnschichtverdampfer (Sambay- oder Luva-Verdampfer) oder als Fallfilmverdampfer ausgelegt sein. Selbstverständlich kann der "Verweilzeit-Verdampfer" ein- oder mehrstufig ausgeführt sein.

Die in der aus der Rektifikationskolonne herausgeführten Rücklaufflüssigkeit in solubilisierter Form angereichert enthaltenen (Meth)acrylsäureoligo- und/oder -polymerisate scheiden sich dabei aus der das hochsiedende hydrophobe organische Lösungsmittel angereichert enthaltenden Sumpfflüssigkeit des "Verweilezeit-Verdampfers" in signifikanten Mengen ab, so daß der dem Abtriebsteil der Rektifikationskolonne zugeführte Ablauf des "Verweilzeit-Verdampfers" eine an (Meth)acrylsäureoligo- und/oder -polymerisaten entreicherte Rücklaufflüssigkeit des Verstärkerteils der Rektifikationskolonne umfaßt, was die Belagsbildung im Abtriebsteil der Rektifikationskolonne signifikant mindert und damit eine verlängerte Betriebsdauer derselben ermöglicht. Selbstverständlich ist von Zeit zu Zeit auch eine Entfernung der im "Verweilzeitverdampfer" auftretenden Belagsbildung erforderlich. Durch Umschaltung auf einen anderen "Verweilzeit-Verdampfer" kann der Betrieb der Rektifikationskolonne jedoch aufrechterhalten werden.

Eine gewisse Belagsminderung im Abtriebsteil der Rektifikationskolonne wird auch dadurch bedingt, daß der aus dem "Verweilzeit-Verdampfer" in den Verstärkerteil der Rektifikationskolonne überführte Brüdendampf die vergleichbare Siedepunkte wie (Meth)acrylsäure aufweisenden niederen Aldehyde ebenfalls angereichert enthält, was eine Verharzung derselben im Abtriebsteil mindert. Die darauf fußende Minderung an Belagsbildung ist jedoch weniger ausgeprägt.

Selbstverständlich wird das erfindungsgemäße Verfahren im Beisein an sich üblicher Mengen von an sich üblichen Polymerisationsinhibitoren durchgeführt. Vorzugsweise wird Phenothiazin als Polymerisationsinhibitor angewendet. Normalerweise werden die Polymerisationsinhibitoren auf die (Meth)acrylsäuremenge (Gewicht) bezogen in Mengen von 50 bis 1000 ppm eingesetzt. Ferner ist es aufgrund der die Polymerisation von (Meth)acrylsäure inhibierenden Wirkung von Luftsauerstoff vorteilhaft, die Rektifikationskolonne luftdurchströmt zu betreiben.

Als Rektifikationskolonne kommen alle gängigen Typen in Betracht. D.h. die Rektifikationskolonne kann z.B. eine Boden-, Füllkörper- oder Packungskolonne sein. Vorzugsweise werden Bodenkolonnen angewendet. Beispielhaft zu nennen sind Ventil-, Glocken-, Tunnel-, Sieb- und Dualflow-Bodenkolonnen. Vorzugsweise werden Glockenböden angewendet. Die Trennlinie zwischen Verstärkerteil und Abtriebteil der Rektifikationskolonne verläuft zweckmäßig etwa am Ende des ersten Drittels der Strecke zwischen unterster und höchstgelegener theoretischer Trennstufe.

Selbstverständlich kann das erfindungsgemäße Verfahren auch mit anderen Verfahren des Standes der Technik zur Unterdrückung von Belagsbildung gekoppelt angewendet werden.

### Beispiele

(Es wurde in Anwesenheit von 200 ppm (bezogen auf das Gewicht der Acrylsäure) Phenothiazin als Polymerisationsinhibitor gearbeitet).
a) Durch katalytische Gasphasenoxidation von Acrolein gemäß Beispiel B1 der DE-A 43 02 991 wurde ein Acrylsäure enthaltendes Reaktionsgasgemisch erzeugt. 2,1 Nm³/1 dieses Reaktionsgasgemisches wurden in einem Gaskühler durch Einspritzen eines Kühlmittelgemisches aus 57,4 Gew.-% Diphenylether, 20,7 Gew.-% Diphenyl und 20 Gew.-% o-Dimethylphthalat auf 170°C gekühlt.
Anschließend wurde in einem Abscheider der flüssig gebliebene Anteil des Kühlmittels von der aus Reaktionsgas und verdampftem Kühlmittel bestehenden Gasphase abgetrennt. Die eine Temperatur von 170°C aufweisende Gasphase wurde unterhalb des ersten Bodens in eine Glockenbodenkolonne mit 27 Böden eines Durchmessers von 80 mm geleitet und dem Gegenstrom von 3 l/h des gleichfalls aus 57,4 Gew.-% Diphenylether, 20,7 Gew.-% Diphenyl und 20 Gew.-% o-Dimethylphthalat zusammengesetzten, am Kolonnenkopf mit einer Temperatur von 45°C aufgegebenen, Absorptionsmittels ausgesetzt. Der Ablauf der Absorptionskolonne wurde in einem Wärmetauscher indirekt auf 105°C erwärmt und auf den Kopf einer Desorptionskolonne gegeben, die als Glockenbodenkolonne mit 20 Böden ausgeführt war. In der Desorptionskolonne wurden im Vergleich zu Acrylsäure leichtsiedende Komponenten wie Essigsäure durch Strippen mit Stickstoff (400 l/h, Gegenstrom) weitgehend aus dem ansonsten Acrylsäure/niedere Aldehyde/Absorptionsmittel enthaltenden Gemisch entfernt. Der Ablauf der Desorptionskolonne bestand aus

| | |
|---|---|
| 84,5 | Gew.-% Absorptionsmittel, |
| 15 | Gew.-% Acrylsäure und |

insbesondere niederen Aldehyden als Nebenkomponenten.
Er wurde mit einer Temperatur von 25°C in einer Menge von 3 l/h zwischen dem fünften und sechsten Boden (vom Verdampfer aus betrachtet) in eine 20 Glockenböden umfassende, luftdurchströmte, Rektifikationskolonne (Durchmesser der Kolonne: 80 mm) geleitet. Die Rektifikationskolonne wurde mit einer Sumpftemperatur von 160°C und einem Sumpfdruck von 130 mbar sowie einem Kopfdruck von 80 mbar betrieben.
Zwischen dem fünf zehnten und sechszehnten Boden (vom Verdampfer aus betrachtet) wurden über Seitenabzug pro Stunde 60 ml Acrylsäure in einer Reinheit von 99,3 Gew.-% kontinuierlich entnommen. Das dampfförmige Kopfprodukt wurde kondensiert, mit Polymerisationsinhibitor versetzt und bis auf eine Entnahmemenge von 5 ml/h oberhalb des obersten Glockenbodens wieder in die Rektifkationskolonne zurückgeführt.
Nach einer Betriebsdauer von 165 h mußte der Betrieb der Rektifikationskolonne infolge zu starker Belagsbildung im Abtriebsteil der Rektifikationskolonne unterbrochen werden.
b) Wie a), der Ablauf der Desorptionskolonne wurde jedoch zunächst in einen einen Volumeninhalt von 500 ml aufweisenden beheizbaren Rundkolben geführt, in selbigem auf eine Temperatur von 150°C erwärmt und bei einer Verweilzeit von 0,5 h und einem Arbeitsvolumen von 250 ml über einen unteren Ablauf des Rundkolbens zwischen dem fünften und sechsten Boden (vom Verdampfer aus betrachtet) der Rektifikationskolonne zugeführt. Nach einer Betriebsdauer von 167 h mußte der Betrieb der Rektifikationskolonne abgebrochen werden. Auf der Innenfläche des Verweilzeit-Rundkolbens war im wesentlichen keine Belagsbildung festzustellen.
c) Wie b), der Verweilzeit-Rundkolben wurde jedoch über ein Brüdenrohr unmittelbar oberhalb des untersten Glockenbodens des Verstärkerteils mit dem Verstärkerteil der Rektifikationskolonne verbunden. Die Betriebsdauer der Rektifikationskolonne bis zum erforderlichen Abbruch verlängerte sich auf 276 h. Auf der Innenfläche des Verweilzeit-Rundkolbens trat mäßige Belagsbildung auf.
d) Wie c), der Abstieg der Rücklaufflüssigkeit vom Verstärkerteil der Rektifikationskolonne in den Abtriebsteil der Rektifikationskolonne wurde jedoch dadurch unterbunden, daß der unterste Boden des Verstärkerteils als Sammelboden gestaltet wurde, von dem die Rücklaufflüssigkeit über eine getauchte Zuführung in den Verweilzeit-Rundkolben geführt wurde.
Auch nach einer Betriebsdauer von 820 h war noch kein Abbruch des Betriebs der Rektifikationskolonne erforderlich. Auf der Innenfläche des Verweilzeit-Rundkolbens trat starke Belagsbildung auf.
Der Gehalt an Acrylsäure im Verweilzeit-Rundkolben betrug im stationären Zustand, bezogen auf das Gewicht der darin enthaltenen Flüssigkeit, 5 Gew.-%.

## Patentansprüche

1. Verfahren der rektifikativen Abtrennung von (Meth)acrylsäure aus einem (Meth)acrylsäure enthaltenden Gemisch, bei dem die (Meth)acrylsäure oberhalb der Stelle, bei der das die (Meth)acrylsäure enthaltende Flüssigkeitsgemisch in die Rektifikationskolonne eintritt, aus der Rektifkationskolonne entnommen wird, und bei dem man wenigstens an einer Stelle der Rektifikationskolonne die in derselben absteigende Rücklaufflüssigkeit aus der Rektifikationskolonne herausführt, dadurch gekennzeichnet, daß man die in der aus der Rektifikationskolonne herausgeführten absteigenden Rücklaufflüssigkeit enthaltene oligo- und/oder polymerisierte (Meth)acrylsäure abtrennt und anschließend die Rücklaufflüssigkeit wieder in die Rektifikationskolonne zurückführt.

## Claims

1. A process for the separation by rectification of (meth)acrylic acid from a mixture containing (meth)acrylic acid, in which process the (meth)acrylic acid is taken off from the rectification column above the point at which the liquid mixture containing the (meth)acrylic acid enters the rectification column and in which process the reflux liquid descending in the rectification column is taken from the rectification column at at least one position on the rectification column, wherein the oligomerized and/or polymerized (meth)acrylic acid present in the descending reflux liquid taken from the rectification column is removed and the reflux liquid is subsequently returned to the rectification column.

## Revendications

1. Procédé de séparation par rectification d'acide (méth)acrylique à partir d'un mélange contenant de l'acide (méth)acrylique, dans lequel on extrait l'acide (méth)acrylique de la colonne de rectification au-dessus du niveau d'alimentation du mélange liquide contenant l'acide (méth)acrylique, et dans lequel on détourne de la colonne de rectification, en au moins un endroit de la colonne de rectification, le liquide de reflux allant vers le haut, caractérisé en ce que l'on sépare l'acide (méth)acrylique, sous forme oligomère et/ou polymère, contenu dans le liquide de reflux allant vers le haut et détourné de la colonne de rectification, puis on renvoie le liquide de reflux dans la colonne de rectification.
